# EUROPEAN PATENT SPECIFICATION

(11) **EP 3 911 730 B1**
(45) Date of publication and mention of the grant of the patent: **08.07.2026**
(21) Application number: 20703890.2
(22) Date of filing: 10.01.2020
(51) Int. Cl.: C12N 5/00

(54) **SYSTEMS AND METHODS FOR CULTURING CELLS IN SUSPENSION**
SYSTEME UND VERFAHREN ZUR KULTIVIERUNG VON ZELLEN IN SUSPENSION
SYSTÈMES ET PROCÉDÉS DE CULTURE DE CELLULES EN SUSPENSION

(30) Priority: 16.01.2019 US 201962793146 P
(43) Date of publication of application: 24.11.2021
(73) Proprietor: Corning Incorporated, Corning, New York 14831 (US)
(72) Inventor: COOPER, Kirsten Lee, Waltham, Massachusetts 02453 (US); WANG, Jie, Weston, Massachusetts 02493 (US); ZUO, Rongjun, West Roxbury, Massachusetts 02132 (US)
(74) Representative: Elkington and Fife LLP
(86) International application number: PCT/US2020/013014
(87) International publication number: WO 2020/150078

(56) References cited:
- WO-A1-2016/200888
- WO-A1-2016/200954
- WO-A2-2014/130864
- ANDRÉ L. RODRIGUES ET AL: "Dissolvable Microcarriers Allow Scalable Expansion And Harvesting Of Human Induced Pluripotent Stem Cells Under Xeno-Free Conditions", BIOTECHNOLOGY JOURNAL, vol. 14, no. 4, 12 November 2018 (2018-11-12), DE, pages 1800461, XP055675245, ISSN: 1860-6768, DOI: 10.1002/biot.201800461
- CHARLIE Y.M. HSU ET AL: "An Integrated Approach toward the Biomanufacturing of Engineered Cell Therapy Products in a Stirred-Suspension Bioreactor", MOLECULAR THERAPY - METHODS & CLINICAL DEVELOP, vol. 9, 1 June 2018 (2018-06-01), GB, pages 376 - 389, XP055675459, ISSN: 2329-0501, DOI: 10.1016/j.omtm.2018.04.007
- FRANKIE MACDONALD: "How Gene Therapy is Becoming a Reality: Overcoming Barriers with Innovative Technologies", 23 April 2019 (2019-04-23), XP055675178, Retrieved from the Internet <URL:https://www.selectscience.net/editorial-articles/how-gene-therapy-is-becoming-a-reality-overcoming-barriers-with-innovative-technologies/?artID=48947> [retrieved on 20200310]

## Description

This Application claims the benefit of priority under 35 U.S.C §120 of U.S. Provisional Application Serial No. 62/793,146 filed on January 16, 2019.

### FIELD OF THE DISCLOSURE

This disclosure general relates to systems and methods for culturing cells. In particular, the present disclosure relates to culturing adherent cells in suspension

### BACKGROUND

The cell culture market is growing at an increasing rate due to rising demand in gene therapy and other applications. However, the yields of currently available culturing systems and methods are not sufficient to meet the demands of commercial-scale applications. In addition, many users of cell culturing systems and methods prefer culturing cells on an adherent platform, due to their familiarity and understanding of their current processes on adherent platforms, and the high yields traditionally achieved from the adherent platform.

Cell culturing is used for growing and harvesting cells and cell products, including viral vectors, such as adeno-associated viral (AAV) vectors. By some estimates, it is believed that the scale necessary for AAV vector commercial applications requires about 10¹⁸ viral titer. Adherent platforms appear to produce high yields of AAV vectors, but challenges remain when scaling-up AAV viral vector production for commercial scale production processes. These challenges include risk of contamination of aseptic steps, processes that are time- and labor-intensive, and lacking in monitoring and control elements, all of which increase not only the complexity but also the expense of AAV viral vector production at a commercial scale.
WO2016200888 discloses digestible substrates for cell culture.
WO2016200954 discloses drying formulation for hydrogel microcarriers.
ANDRE L. RODRIGUES ET AL: BIOTECHNOLOGY JOURNAL, vol. 14, no. 4, 12 November 2018, page 1800461 discloses that Dissolvable Microcarriers Allow Scalable Expansion And Harvesting Of Human Induced Pluripotent Stem Cells Under Xeno-Free Conditions.
WO2014130864 discloses microcarrier perfusion culturing methods and uses thereof. CHARLIE Y.M. HSU ET AL: MOLECULAR THERAPY - METHODS & CLINICAL DEVELOP, vol. 9, 1 June 2018 pages 376-389 discloses An Integrated Approach toward the Biomanufacturing of Engineered Cell Therapy Products in a Stirred Suspension Bioreactor.

### SUMMARY

In embodiments, the present disclosure provides a method of culturing adherent cells in suspension according to claim 1

### BRIEF DESCRIPTION OF THE DRAWINGS

Figure 1 shows a traditional method of culturing adherent cells on an adherent platform which is not an embodiment of the present disclosure.
Figure 2 shows a method of culturing adherent cells in suspension, according to one or more embodiments of the present disclosure.
Figure 3 shows cell yield using microcarriers in suspension compared to a 2D substrate, according to an example of one or more embodiments.
Figure 4A shows a comparison of viral genome (VG) number (qPCR titer) for adherent and suspension cells using adherent and suspension platforms in terms of viral genome per sample.
Figure 4B shows a comparison of viral genome (VG) number (qPCR titer) for adherent and suspension cells using adherent and suspension platforms in terms of viral genome per cell.
Figure 5A shows the infectious titer measurements of the example in Figure 4A.
Figure 5B shows the infectious titer measurements of the examples in Figure 4B.
Figure 6A shows a micrograph image of microcarriers before dissolution, according to one or more embodiments.
Figure 6B shows a micrograph image of microcarriers during dissolution, according to one or more embodiments.
Figure 6C shows a micrograph image of microcarriers after dissolution, according to one or more embodiments.
Figure 7A is a graph showing the viral genome per sample of adherent cells recovered on various surfaces.
Figure 7B is a graph showing the viral genome per cell of adherent cells recovered on various surfaces.
Figure 8A is a graph showing comparisons of the infectious titers per samples of adherent cells recovered on various surfaces.
Figure 8B is a graph showing comparisons of the infectious titers per cell for adherent cells recovered on various surfaces
Figure 9 shows a comparison of cell yield after using a post-electroporation dissolution process of the present disclosure as compared to a standard post-electroporation dissolution process.

### DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not limiting and merely set forth some of the many possible embodiments of the claimed invention.

Embodiments of this disclosure include methods of cell culturing for growing and harvesting cells and cell products, including viral vectors, such as adeno-associated viral (AAV) vectors. In particular, one or more embodiments include methods for culturing adherent cells on substrates in suspension. Adherent platforms appear to produce higher yields of AAV vectors, possibly as high as 10-fold or greater for AAV genome particles and infectious titer as compared to suspension cells under the same transfection conditions. But unlike adherent platforms, suspension platforms offer advantages for scalability. The systems and methods disclosed herein enable scaling-up of adherent cell culturing by culturing in suspension to achieve greater viral loads upon harvest, including up to and exceeding about 10¹⁸ viral titer. Previously, switching to a suspension platform might require users to develop a new cell line or modify a current cell line for suspension mode culturing, which may result in a lower titer yield, and it might also require developing new media, such as chemically defined serum-free media, that is compatible with cell growth and current transfection reagents, such calcium phosphate (CaPi) or polyethylenimine (PEI). However, these adjustments are often disfavored by users.

According to embodiments herein, microcarriers can be used to grow adherent cells in suspension, and those microcarriers can be dissolved at the time of harvesting. Thus, many of the challenges associated with switching to a suspension platform are avoided, but the scalability of a suspension platform can be achieved. In addition, embodiments herein use electroporation in combination with in-suspension microcarrier cell substrates. The methods disclosed herein combines the advantages of adherent cells with the advantages of suspension cell platforms in commercial-scale cell culturing applications. (e.g., AAV viral vector production process). The advantages include high cell product (e.g., AAV viral vector) yield, user satisfaction and ease of operating due to their understanding of the cell line and medium, process scalability, cost, easy handling, and minimized risk of cell contamination.

### Definitions

"Wholly synthetic" or "fully synthetic" refers to a cell culture article, such as a microcarrier or surface of a culture vessel, that is composed entirely of synthetic source materials and is devoid of any animal derived or animal sourced materials. The disclosed wholly synthetic cell culture article eliminates the risk of xenogeneic contamination.

"Include," "includes," or like terms means encompassing but not limited to, that is, inclusive and not exclusive.

"Users" refers to those who use the systems, methods, articles, or kits disclosed herein, and include those who are culturing cells for harvesting of cells or cell products, or those who are using cells or cell products cultured and/or harvested according to embodiments herein.

"About" modifying, for example, the quantity of an ingredient in a composition, concentrations, volumes, process temperature, process time, yields, flow rates, pressures, viscosities, and like values, and ranges thereof, or a dimension of a component, and like values, and ranges thereof, employed in describing the embodiments of the disclosure, refers to variation in the numerical quantity that can occur, for example: through typical measuring and handling procedures used for preparing materials, compositions, composites, concentrates, component parts, articles of manufacture, or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture.

"Optional" or "optionally" means that the subsequently described event or circumstance can or cannot occur, and that the description includes instances where the event or circumstance occurs and instances where it does not.

The indefinite article "a" or "an" and its corresponding definite article "the" as used herein means at least one, or one or more, unless specified otherwise.

Abbreviations, which are well known to one of ordinary skill in the art, may be used (e.g., "h" or "hrs" for hour or hours, "g" or "gm" for gram(s), "mL" for milliliters, and "rt" for room temperature, "nm" for nanometers, and like abbreviations).

Specific and preferred values disclosed for components, ingredients, additives, dimensions, conditions, and like aspects, and ranges thereof, are for illustration only; they do not exclude other defined values or other values within defined ranges. The systems, kits, and methods of the disclosure can include any value or any combination of the values, specific values, more specific values, and preferred values described herein, including explicit or implicit intermediate values and ranges.

The methods disclosed herein are applicable to different cells lines. In one or more embodiments, adherent HEK293 cells are used, including, for example, HEK293T cells, which have been found to effectively grow and culture on dissolvable microcarriers. References to HEK293 or HEK293T in the embodiments and examples discussed herein are understood to be illustrative examples, and are not intended to limit the scope of these embodiments to a particular cell type. Any type of cell may be cultured according to the embodiments herein including, but not limited to, immortalized cells, primary culture cells, cancer cells, stem cells (e.g., embryonic or induced pluripotent), etc. The cells may be mammalian cells, avian cells, piscine cells, etc. The cells may be of any tissue type including, but not limited to, kidney, fibroblast, breast, skin, brain, ovary, lung, bone, nerve, muscle, cardiac, colorectal, pancreas, immune (e.g., B cell), blood, etc. The cells may be in any cultured form including disperse (e.g., freshly seeded), confluent, 2-dimensional, 3-dimensional, spheroid, etc. Culturing cells on a substrate, such as a dissolvable microcarrier, may include seeding cells on the substrate, which may include contacting the substrate with a solution containing the cells. During seeding cells on the substrate, the cells may adhere to a surface substrate, including enter into pores of the substrate. Where the substrate is a microcarrier including an adhesion polymer coating, cells may enter the pores of the microcarrier and attach to the scaffold material.

One or more embodiments are directed to culturing cells and harvesting cells or cell products, including viral vectors or proteins. In particular embodiments, for example, methods are directed to generating and/or harvesting AAV viral vectors or lentivirus vectors. References to AAV viral vectors in the embodiments and examples discussed herein are understood to be illustrative examples, and are not intended to limit the scope of these embodiments to a particular cell culture application. For example, embodiments include the generation of other viral vector, proteins, and cell products, including lentivirus production, for example.

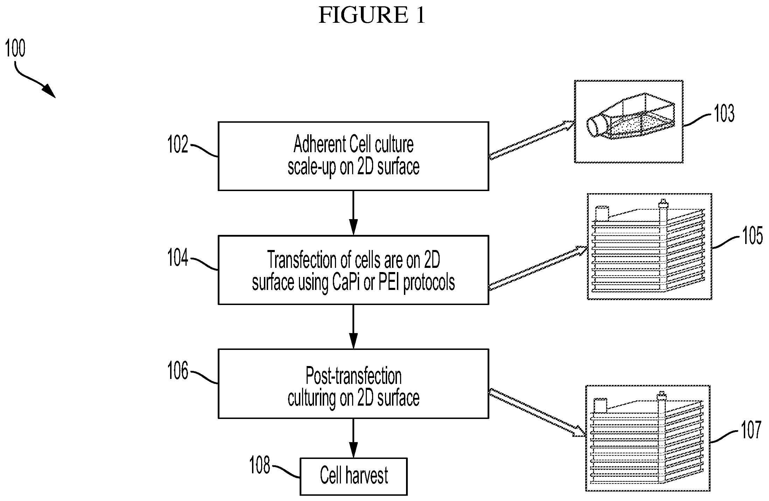

Aspects of embodiments of this disclosure can be appreciated with respect to difference between traditional methods of AAV viral vector production methods and the methods of this disclosure. Traditionally, AAV viral vector production occurs in an adherent platform where cells are grown on a two-dimensional (2D) surface. For example, as shown in a traditional production process 100 illustrated in Figure 1, in Step 102, adherent cells (such as HEK-293Tcells) are scaled-up using 2D surfaces (e.g., a flask 103). Next, in Step 104, transfection occurs using PEI or CaPi methods on cells attached to the 2D surfaces (e.g., such as a HYPERstack^{®} flask or other layer vessel 105, 107), and the cells stay on the 2D surfaces for post-transfecting culturing (Step 106). Finally, in Step 108, the cells or cell products are harvested from the culture surface or medium. Challenges facing the adherent platform method in Figure 1 include scalability, labor and time intensive nature, high risk of contamination, large footprint of 2D culture surfaces requiring large equipment and lab space, difficulty in managing due to size, lacking control in the process, and the process being costly.

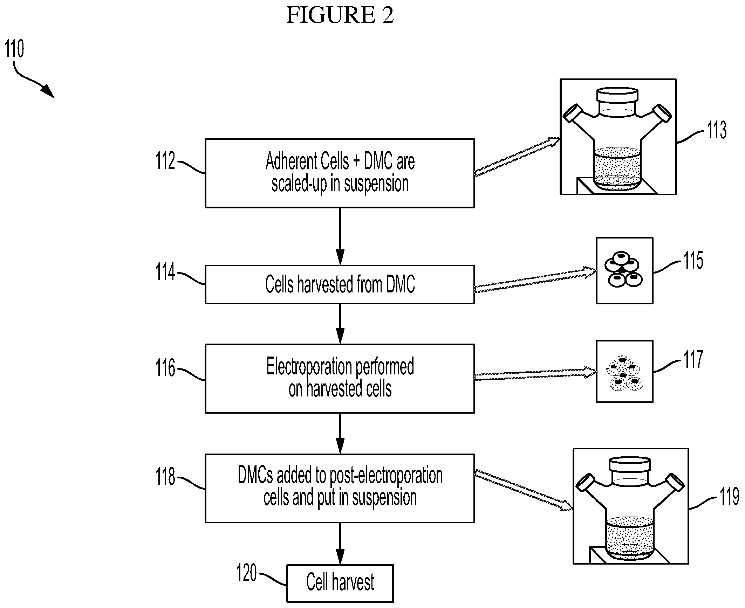

In contrast, according to one or more methods of this disclosure, adherent cells (such as HEK293) are scaled-up in suspension using dissolvable microcarrier (DMC) substrates, as shown in the process 110 of Figure 2. This scale-up (Step 112) can be performed in a three-dimensional (3D) volume 113, such as a spinner flask or bioreactor. After scale-up, at Step 114, the cells 115 may be harvested from the DMC substrates so that transfection can be performed in step 116 using electroporation. After electroporation, the transfected adherent cells 117 are recovered on DMC substrates or another suspension format for post-transfection culturing (Step 118; see vessel 119), and are later harvested (Step 120).

By switching the adherent cell culture vessels from traditional 2D surfaces to in-suspension dissolvable microcarriers during cell culture scale-up (Step 112 in Figure 2), the cell yield increases. Specifically, a 1.5- to 2-fold increase in cell yield has been achieved, when using the same amount of culturing time. In addition, the cell functions remain the same, cell culture handling is easier, laboratory footprint is decreased, and the risk of contamination is minimized. Viral genome titration and infectious titer titration indicates no difference between cells scaled up from DMC or from 2D surfaces. The electroporation step (Step 116) has the advantages of high transfection efficiency, ease of scale-up, batch-to-batch consistency, and time saving. For example, time can be reduced by up to about 24 hours or more in a post-transfection expression stage. In the post-transfection culture recovery (Step 118), the same benefits can be achieved as discussed above for Step 112.

The culturing of cells on microcarriers can be carried out in a bioreactor or similar vessel. During culturing, the cells grow on the surface of the microcarriers. Once the cell culturing process is completed, the cultured cells are detached from the microcarriers and the cultured solution containing the cells is then separated from the microcarriers for use or further processing. Aspects of embodiments of this disclosure include adding cells and cell growth medium to a vessel of a bioreactor system and adding dissolvable microcarriers to the vessel. Additionally, the method includes dissolving the dissolvable microcarriers, and removing spent medium with dissolved microcarrier material from the vessel. Separating the microcarriers from the cultured solution that includes the detached cells can be performed using a number of techniques, including, for example, differential gradient centrifugation, acoustic resonance, tangential flow filtration, filtration through a mesh screen, spin filters, and sedimentation using conical or inclined plates.

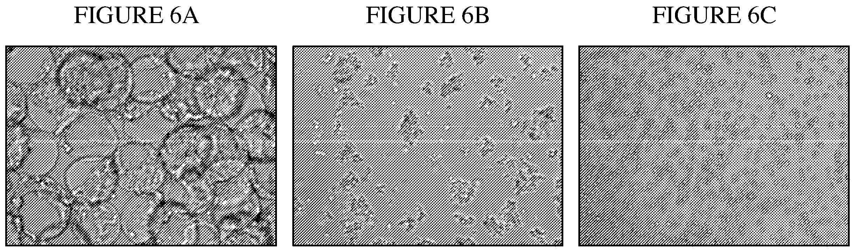

According to one or more embodiments, cells that are harvested (e.g., at step 114 of Figure 2) are transfected using electroporation (EP) (e.g., step 116 of Figure 2). This overcomes the challenges encountered with using traditional methods of transfection using PEI and CaPi methods, which do not work efficiently for cells attached to microcarriers. Electroporation requires single-cell suspension, which is made possible by harvesting cells from the microcarriers of this disclosure. In addition, the electroporation procedure provides the benefits of being a well-controlled process, having high and consistent performance, saving time in viral vector expression (48 hours for electroporation versus 72 hours for PEI or CaPi), and cost efficiency. Figures 6A-6C show the progression from cultured cells on microcarriers (Figure 6A), dissolving of the microcarriers within about 3 to 4 minutes of contacting with the digesting enzymes or agents (Figure 6B), and the suspension of single cells after resuspension in the medium (Figure 6C).

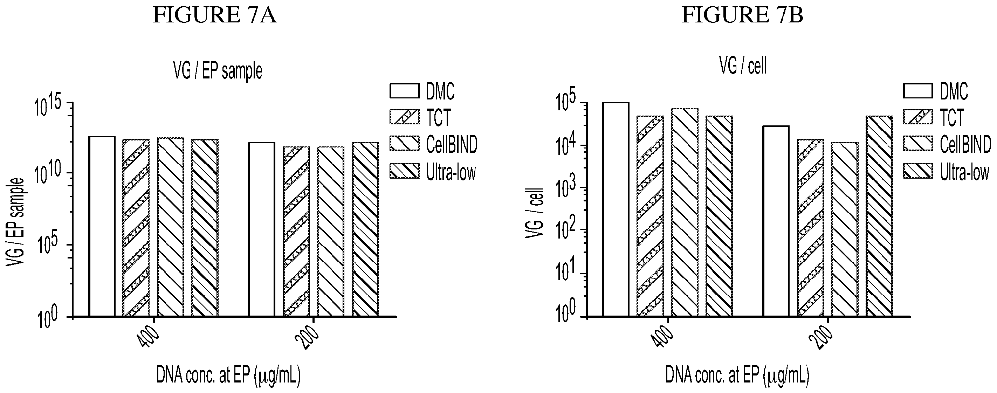

Following electroporation, the adherent cells are recovered on microcarriers. Traditionally, adherent cells are recovered in 2D surfaces for post-transfection culturing, even when using electroporation for transfection. However, doing so may eliminate the benefits from the previous steps in the embodiments. For example, the cells would need to go back to an adherent platform with all the disadvantages (such as large space requirements, etc.). Thus, the disclosure includes recovery using microcarriers. Results from examples of such embodiments show that adherent cells do not require 2D surfaces for recovery, Microcarriers and other suspension formats (e.g. spinner flask, shake flask, etc.) performs as well as or better than 2D surfaces. Examples of the viral genome number for cells recovered on different substrates, see Figures 7A and 7B, which are discussed below. Thus, cells can be recovered into the suspension format.

As an aspect of one or more embodiments, a new microcarrier dissolution process is provided when using microcarriers as a post-electroporation substrate. That is, a new dissolution process for dissolving the microcarriers used in the post-electroporation suspension for harvesting the cells or cell products. The standard dissolvable microcarrier dissolution process involves three steps: 1) washing the microcarriers to remove residual serum; 2) dissolving the microcarriers with a solution containing one or more enzymes and/or chelators, such as a combination of TrypLE, pectinase, and EDTA; and 3) after microcarrier digestion, washing the cells to remove residual TrypLE and pectinase. The wash in step 1 prevents the serum from interfering with the TrypeLE and pectinase digestion in step 2. The wash in step 3 eliminates the potential negative impact of the enzymes in downstream processing. For using dissolvable microcarriers according to one or more embodiments of this disclosure, a new simplified dissolution process was developed.

According to one or more embodiments, this new dissolution process may be used when microcarriers are used for recovering cells post-electroporation. This dissolution process may include added a chelator, such as EDTA, directly to the cell culture medium, waiting a predetermined time, and then proceeding to isolate and/or collect the cells or cell products. The predetermined time can be, for example, about 1 hour or less, about 30 minutes or less, or about 10 minutes, or from about 1 minute to about 30 minutes. This dissolution process is effective when the target of harvesting is AAV viral vectors or lentivirus production.

The dissolution process can have no wash steps, can be performed preferably with no protease (e.g. TrypLE, Trypsin) or other enzymes (e.g. pectinase), and has high digestion efficiency to dissolve all microcarriers in a short period of time (e.g., in about 30 minutes or less, or in about 10 minutes). The removal of all microcarriers is helpful because of the relatively large volume of the microcarriers and the ability of them to interfere with the downstream steps, such as cell lysis and purification processes.

The post-electroporation dissolution process benefits from having no wash steps. At the time of harvest, a portion of the cells may be detached from the dissolvable microcarriers due to the expression of viral vectors or other cell products. To collect both the cells/cell products attached to the microcarriers and the cells/cell products in the medium, a higher centrifugation speed and time may be used. However, the combination of high centrifugation speed and/or long centrifugation time, large volume of microcarriers, and/or loose attachment of cells to microcarriers, makes washing steps difficult to operate and can lead to cell loss and viral titer loss. In addition, if users want to collect viral vectors in the cell culture medium, washes will increase supernatant volume, which increases time and labor involved, and causes loss of viral vector in the wash waste.

The post-electroporation dissolution process also benefits from no requiring the use of enzymes such as protease (e.g., TrypLE, Trypsin), pectinase, or other enzymes. Protease can cleave cell products, such as AAV viral vectors, thus decreases the infectivity of the harvested viral vectors. If protease has to be used, it will become critical to minimize time of exposure and wash the cells afterwards to assure functionality of the viral vectors.

It is noted that one or more embodiments of this disclosure include a cell culturing method in which pre-electroporation microcarriers can be dissolved using the standard dissolution process (e.g., the three-step process washing and adding enzymes including protease and/or pectinase, as discussed above, while the post-electroporation microcarriers can be dissolved according to the new and streamlined dissolution process discussed above. This new dissolution process has not previously been used for cells in suspension, and proves to be an unexpectedly efficient process that does not require the problematic enzymes and washes of the standard process.

Trypsin is frequently applied to dissociate adhesive cells from the substratum once cultured cells reach confluence. For example, U.S. Pat. No. 4,994,388, discloses a method for culturing and harvesting anchorage-dependent cells employing microcarrier beads coated with collagen. Once growth is complete, the collagen is digested off of the microcarrier, and the cultured cells are separated from the insoluble microcarrier. However, due to the proteolytic activity of trypsin, cell surface proteins are often cleaved, which may lead to dysregulation of the cell functions. It has been reported that trypsin is able to induce proteome alteration and cell physiological changes. Huang, et al., reported that trypsinization down-regulated growth- and metabolism-related protein expressions and up-regulated apoptosis-related protein expressions, implying that trypsin used for cell subculture had an adverse effect on cell physiology (see Hsiang-Ling Huang et al., "Trypsin-induced proteome alteration during cell subculture in mammalian cells", Journal of Biomedical Science, 2010, 17:36.).

As another example of deleterious effect of proteases, it is also known that treatment of cells with proteases such as trypsin remove antigens from cancer cells and might make them unusable to develop vaccines for anti-cancer therapies. Lokhov, et al., reported that trypsinization releases glycoproteins and sugars from the cell surface (see "A sialomucopeptide liberated by trypsin from the human erythrocyte," Nature, 1960; 188:1011-2, Gasic, G. "Removal and regeneration of the cell coating in tumour cells," Nature, 1962; 196:170, and Uhlenbruck, G., in "Action of proteolytic enzymes on the human erythrocyte surface," Nature, 1961; 190:181 and in "The isolation of living cells from animal tissues," International Review of Cytology, 1956; 7:587-647), thereby leading to a loss of antigenic properties (see David, J.R., et al., "The In Vitro, Desensitization of Sensitive Cells by Trypsin," J Exp Med., 1964;120:1189-200, 40, Weiss, L, et al., "The demonstration of rupture of cell surfaces by an immunological technique," Exp Cell Res., 1963; 30:331-8, Osunkoya, B.O., et al., "Synthesis and fate of immunological surface receptors on cultured Burkitt lymphoma cells," Int J Cancer, 1969; 4(2):159-65, Takeichi, N, et al., "Accelerated regeneration of trypsin-treated surface antigens of simian virus 40-transformed BALB/3T3 cells induced by X-irradiation," Cancer Res., 1976; 36(4):1258-62). In particular, Takeichi, et al., showed that trypsin treatment of SV40-transformed 3T3 cells decreased their antigenicity in footpad assays. Similarly, it has been demonstrated that polyoma virustransformed cells treated with trypsin failed to induce a delayed hypersensitivity reaction against tumor-specific antigens in footpad swelling assays (see Molinari, J.A., et al., "Modification of surface membrane antigens by trypsin," Proc Soc Exp Biol Med., 1975; 148(4):991-4).

U.S. Pat. No. 5,100,799, Mundt, et al., discloses a method for releasing cell cultures from microcarriers in which a trypsin solution is directed through a container with the microcarriers in a flow-through process. The patent mentions that the released cells must be immediately withdrawn from the carrier, with the trypsin solution being inactivated, removed, or both, after leaving the container to prevent deleterious effect of the protease on the harvested cells. Mundt mentioned that a "high percentage of the cells is released rather quickly and therefore remains in the trypsin solution for a long time. This results in the disadvantage that the action of the trypsin exerts an adverse influence on these cells. Cell growth and cell resettlement capability on new microcarriers that is particularly adversely affected.

As follows from the above, harvesting cells without trypsin or in the absence of trypsin is highly desirable. Pectinases are another enzyme used for carrier digestion. Pectinases (polygalacturonase) are enzymes that break down complex pectin molecules to shorter molecules of galacturonic acid. Pectinase catalyzes the liberation of pectic oligosaccharides (POS) from polygalacturonic acid.

Systems and methods that enable cell expansion in serum free condition, in a chemically defined medium, and that allow cell harvesting without adding protease (e.g., TrypLE, trypsin, etc.) or other enzymes (e.g., pectinase), as disclosed in the present application would be useful.

The method is disclosed according to claim 1.

After electroporation, the cells are recovered on a second substrate in suspension. The second substrate may includes a second microcarrier that may be substantially the same as the first microcarrier, or may be a different type of microcarrier.

According to the present disclosure, the method comprises a dissolution process for dissolving the second dissolvable microcarrier particle to harvest the cells or cell products. The dissolution process may include adding a chelator to the second suspension; contacting the cultured cells with the chelator for a predetermined time to separate the cells from the second microcarrier; and isolating the cells or cell products from a remainder of the second suspension after the predetermined time. The chelator can be ethylenediamine tetraacetic acid (EDTA), or a suitable alternative. The predetermined time may be from about 1 minute to about 30 minutes; from about 5 minutes to about 20 minutes, from about 8 minutes to about 12 minutes, or at least about 10 minutes.

In one or more embodiments, the contacting and the separation of the cells from the second microcarrier are performed free of a protease, a pectinase, or both a protease and a pectinase. In addition, the dissolution process may not include a washing step, such as a phosphate-buffered saline wash.

The isolating of the cells or cell products can include centrifuging or filtering the second suspension to separate the cells or cell products from the remainder of the second suspension. The method also includes harvesting the cells or products of the cells from the second suspension. Lysis of the isolated cells or cell products can also be performed.

As an aspect of some embodiments, the transfected cells in the second suspension produce adeno-associated virus (AAV) or lentivirus viral vectors for harvesting.

Although not limited to specific cells for culturing, the cells may be adherent HEK293 cells, such as HEK293-T cells.

The culturing step may include the first suspension or the second suspension being contained in a bioreactor, a shake flask, a spinner flask, or other suspension vessel.

As an aspect of one or more embodiments, the electroporation is performed for about 48 hours or less, about 24 hours or less, about 12 hours or less, about 6 hours or less, about 4 hours or less, about 3 hours or less, about 2 hours or less, about 1 hour or less, about 30 minutes or less, about 10 minutes or less, or about 10 minutes to about 1 hour.

The first microcarrier particle and/or the second microcarrier particle can include a base substrate having at least one of a polygalacturonic acid compound selected from at least one of: pectic acid; partially esterified pectic acid, partially amidated pectic acid, or salts thereof. The partially esterified pectic acid may include a degree of esterification from 1 to 40 mol%. The first microcarrier particle and/or the second microcarrier particle may also include at least one first water-soluble polymer having surface activity, or a water soluble plasticizer. The polygalacturonic acid compound can be covalently cross-linked, ionically cross-linked, or mixtures thereof. The partially esterified pectic acid can include an alkyl carboxy ester having an alkyl group having from 1 to 10 carbon atoms.

In some embodiments, the first microcarrier particle or the second microcarrier particle further comprises an adhesion polymer on the surface of the polygalacturonic acid compound. The adhesion polymer on the surface of the polygalacturonic acid compound can include a peptide or a polypeptide. The adhesion polymer coating can also include peptides selected from the group consisting of BSP, vitronectin, fibronectin, laminin, Type I collagen, Type IV collagen, denatured collagen and mixtures thereof. The adhesion polymer may promote the attachment of anchorage dependent live cells to the substrate.

One or more embodiments of this disclosure include a method of producing adeno-associated virus (AAV) viral vectors using the method of culturing adherent cells in suspension as discussed in the embodiments above, where the AAV viral vectors are harvested from the cultured cells or from the culture medium.

The method is disclosed according to claim 1. The harvesting can include contacting the cultured cells in the culturing suspension with a chelator, such as EDTA, to separate the cells from the microcarrier.

In another embodiment, a method for harvesting cultured cells is provided that includes providing an adherent cell on a microcarrier particle in suspension; and contacting the cultured cell with a chelator to separate the cells from the microcarrier particle. The separated cells may be isolated from the composition to harvest the cells or cell products. As an aspect of one or more embodiments, the contacting of the microcarrier with the chelator is accomplished free of a protease, a pectinase, or both a protease and a pectinase.

### Examples

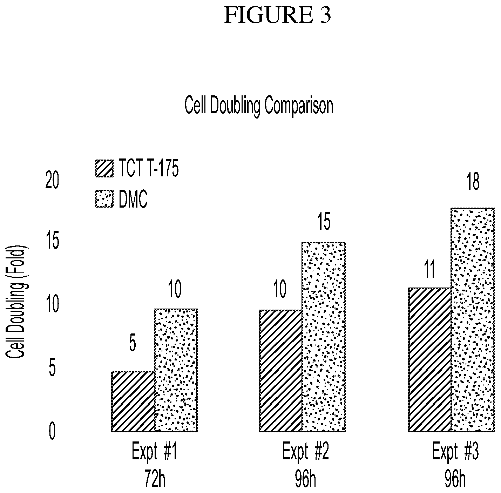

As discussed above, according to embodiments of this disclosure, a 1.5- to 2-fold increase in cell yield has been achieved, when using the same amount of culturing time. This increase in cell yield is illustrated in Figure 3, where the cell yield resulting from culturing adherent cells in suspension on DMCs is compared to the cell yield from culturing adherent cell on a 2D surface in a TFT T-175 flask in three experiments. In each of Experiments #1-#3 of Figure 3, the same number of cells (10e6) were seeded, with similar incubator footprints, and cultured for the same culture time was used for each type of substrate within each experiment. Experiment 1 shows the yield after 72 hours of culturing on the respective surfaces, and experiments 2 and 3 show the yield after 96 hours. The cell recovery from both substrate types (DMC and 2D flask) was greater than 95%. However, the yields from using the DMC substrate in suspension are 1.5- to 2-times greater than that of the 2D surface.

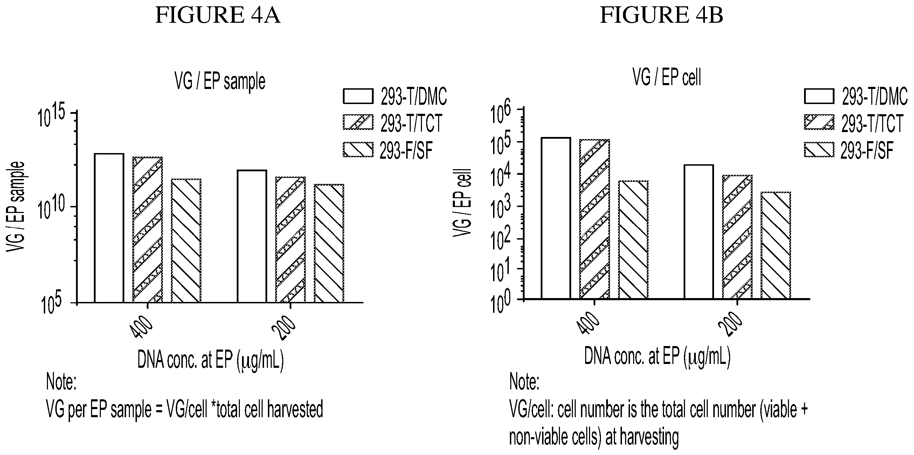

Figures 4A and 4B illustrate the comparable viral genome number (qPCR) measured from adherent 293-T cells cultured on a 2D surface in the TCT flask and cultured on DMCs according to embodiments of this disclosure. In addition, these values of the infectious titer are compared to that from suspension-type 293-F cells. The results show an increase (an average about 10-fold) in infectious titer for the adherent 293-T cells as compared to the suspension 293-F cells. The cells were transfected using electroporation (EP).

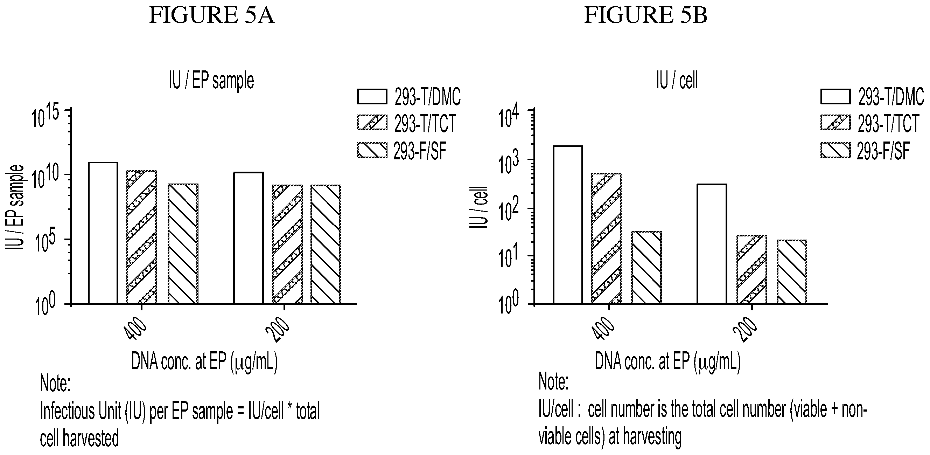

In Figures 5A and 5B, the infection titer (measured by Taqman TCID₅₀) was compared between the samples from Figures 4A and 4B, and shows a similar trend as that shown in Figures 4A and 4B. Specifically, the adherent 293T cells yielded more infectious titer than suspension 293-F cells, with an average of about a 10-fold increase, and the 293T cells that were scaled-up using DMCs showed comparable competency as the 293T cells scaled-up using the TCT surface.

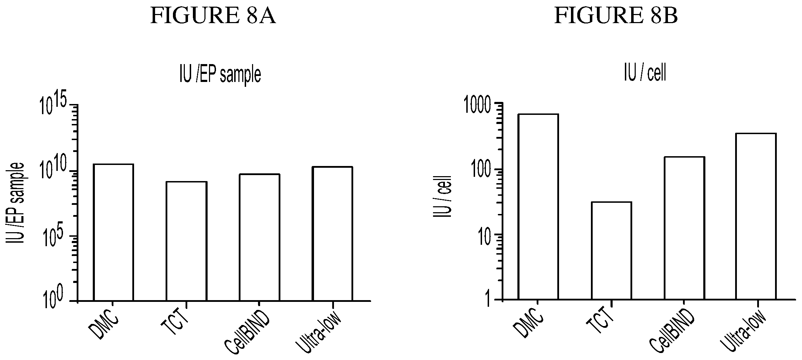

In Figures 7A and 7B, the viral genome numbers for cells recovered on different substrates are compared. As shown in Figures 7A and 7B, the 293T cells recovered on four different substrates (dissolvable microcarriers, TCT flask, CellBind flask, and Ultra-low attachment flask) showed comparable AAV viral genomes per EP sample and per cell (the difference between samples was less than two-fold). In Figures 8A and 8B, the infectious titer achieved from the different recovery surfaces is compared, based on an electroporation (EP) DNA concentration of 400 µg/ml. In these examples, the dissolvable microcarriers and ultra-low surface flask provided the best infectious titer in post-electroporation recovery. The method of the present disclosure requires the use of a second dissolvable microcarrier, thus the examples which do not use a second dissolvable microcarrier are not embodiments of the present disclosure.

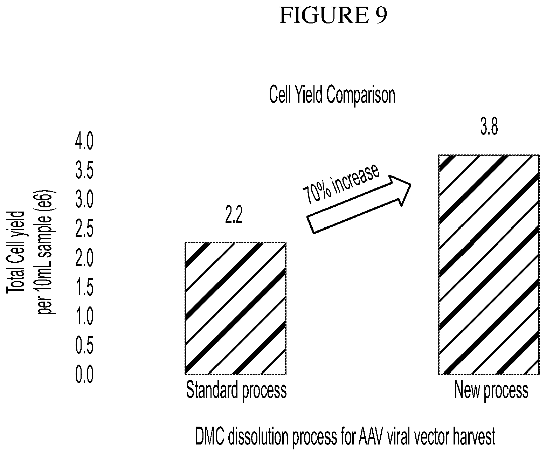

In Figure 9, the cell yield results for a process using a standard post-electroporation dissolution process are compared to those of a process using the new post-electroporation dissolution process of this disclosure. As shown in Figure 9, the new process resulted in a total cell yield of about 3.8e6 per 10 ml sample, compared to 2.2e6 per 10ml sample from the standard dissolution process. That is, the dissolution process of the present disclosure resulted in a 70% increase in total cell yield. The viral vector yield improves proportionally to this cell yield increase.

### Cell Culture Substrate

The host cell cultures of the present disclosure may be cultured on a suitable culture substrate. In one or more embodiments, the substrate is in suspension, and the substrate may be a so-called microcarrier. Suitable microcarrier articles and methods of making are described in U.S. Patent Application Publication Nos. US20130071916 and US20160145567, as well as U.S. Provisional Patent Application 62/632178. In some embodiments, the substrate may be a polygalacturonic acid (PGA) microcarrier coating with an adhesion polymer.

The microcarrier can enable rapid and complete cell harvesting by contacting the microcarrier, on which the cells were grown, with pectinase, and optionally a chelating agent, e.g., ethylenediamine tetraacetic acid (EDTA), and without the need of adding any protease. However, according to the methods disclosed herein, the use of the microcarrier can further enable rapid and complete cell harvesting by contacting the microcarrier, on which the cells were grown, with only a chelating agent, such as EDTA, and neither protease nor pectinase need to be added. Thus, according to the embodiments of this disclosure, the use of only a chelating agent, such as EDTA, without the need for protease and pectinase, is unexpectedly effective and efficient at harvesting cells from the microcarrier, and reduces the number of steps and materials (e.g., enzymes such as protease or pectinase) needed to complete the harvest. In embodiments, the chelator can be, for example, EDTA, like multi dentate chelators, or mixtures thereof.

According to one or more embodiments, a method for coating a surface of a cell culture article includes dissolving a polymer having a covalently attached polypeptide in an aqueous solution to produce a polymer solution. The polymer is formed from monofunctional monomers (i.e., no monomers having di- or higher- functionality). The weight percentage of the polypeptide relative to the polymer conjugated to the polypeptide is sufficiently high to render the polymer conjugated to the polypeptide water soluble. The aqueous solution is substantially free of organic solvents. The method further includes (i) disposing the polymer solution on the surface of the cell culture article to produce a coated article; and (ii) subjecting the coated article to sufficient heat or electromagnetic radiation to attach the polymer conjugated to a polypeptide to the surface of the cell culture article. Examples of suitable peptide conjugated polymers include, for example, p(MAA-PEG₄-VN) and p(HEMA-co-MAA-PEG₄-VN), or mixtures thereof (*see* Figures 9 and 10 of US20160145567). In the foregoing, "MAA" is methacrylic acid, "HEMA" is hydroxyethylmethacrylate, "PEG₄" is a polyethylene glycol tetra oligomer, and "VN" is a conjugated vitronectin polypeptide.

The synthetic microcarriers of the disclosure may be prepared, for example, from at least an ionotropically cross-linked biopolymer selected, for example, from: pectic acid (also known as PGA); or partially esterified pectic acid (also known as pectinic acid) (PE PGE); or salts thereof, or a mixture thereof. When partially esterified pectic acid is selected, the degree of esterification can be, for example, about 40 mol% or less, such from 1 mol% to 39 mol%, 5 to 35 mol%, 10 to 30 mol%, including intermediate values and ranges.

In embodiments, the microcarrier of the disclosure is advantageously and preferably made of pectic acid or pectinic acid beads, or salts thereof, having microsphere dimensions, which beads are functionalized with cell adhesion promoting peptides, and more preferably functionalized by coating with a synthetic polymer bearing adhesion peptides. The adhesion peptides enable bio-specific adhesion of the cultured cells. The disclosed microcarrier is suitable for large-scale expansion and recovery of adherent cells in serum free culture.

The cell culture substrate composition can comprise, for example, at least pectic acid, partially esterifed pectic acid, or salts thereof, and mixtures thereof; and at least one peptide promoting the attachment of anchorage dependent cells.

Preferably the composition comprises a peptide-polymer conjugate, which conjugate promotes the attachment of anchorage dependent cells, more preferably the peptide-polymer conjugate is poly (meth)acrylate or poly(meth)acrylamide copolymer comprising an adhesion peptide, and more preferably the peptide-polymer conjugate is Synthemax^{®}II.

The synthetic microcarrier of the disclosure can be made of at least one ionotropically cross-linked polysaccharide selected from, for example, pectic acid, also known as polygalacturonic acid (PGA), or a salt thereof, or partly esterified pectic acid (PE PGA) known as pectinic acid, or a salt thereof. When pectinic acid is selected, the degree of esterification is preferably less than about 40 mol% since a higher degree of esterification makes bead formation by ionotropic crosslinking ineffective. Without being bound by theory it is believed that a minimum amount of free carboxylic acid groups may be called for to obtain an acceptable level of ionotropic crosslinking. The crosslinking may be obtained by an ionotropic gelation method, and including being obtained by at least one internal gelation method.

The beads used as the microcarrier of the disclosure may be prepared from a mixture of pectic acid or pectinic acid. Pectic acid can be formed by the hydrolysis of certain esters of pectins. Pectins are cell wall polysaccharides which have a structural role in plants. They are predominantly linear polymers based on a 1,4-linked alpha-D-galacturonate backbone, interrupted randomly by 1,2-linked L-rhamnose. The average molecular weight is from about 50,000 to about 200,000 Daltons.

Two major sources of pectins are, for example, from citrus peel (mostly lemon and lime) or apple peels, and can be obtained by extraction thereof.

The polygalacturonic acid chain of pectins can be partly esterified with methyl groups and the free acid groups may be partly or fully neutralized with monovalent ions such as sodium, potassium, or ammonium ions. Polygalacturonic acids partly esterified with methanol is called pectinic acids, and salts thereof are called pectinates.

The degree of methylation (DM) for commercial high methoxyl (HM) pectins typically can be, for example, from 60 to 75 mol% and those for low methoxyl (LM) pectins can be from 1 to 40 mol%, 10 to 40 mol%, and 20 to 40 mol%, including intermediate values and ranges.

The microcarriers of the disclosure are preferably prepared from the LM pectins and preferably the polygalacturonic acid contains less than 20 mol% methoxyl groups, and more preferably the polygalacturonic acid has no or only negligible methyl ester content as pectic acids. For simplicity both pectinic acid having no or only negligible methyl ester and low methoxyl (LM) pectins are referred to as PGA in the disclosure.

The PGA beads can be functionalized with moieties promoting cell adhesion, for example, with peptides. Peptides containing amino acid sequences potentially recognized by proteins from the integrin family, or leading to an interaction with cellular molecules able to sustain cell adhesion, are candidates for functionalizing the present microcarriers. Preferred peptides can be, for example, selected from BSP, vitronectin, fibronectin, laminin, collagen, and like peptides, and mixtures thereof. Particular example peptides are vitronectin (VN) and BSP peptides having the following sequences: Ac-Lys-Gly-Pro-Gln-Val-Thr-Arg-Gly-Asp-Val-Phe-Thr-Met-Pro-NH₂ (seq. ID No.: 1), and Ac-Lys-Gly-Gly-Asn-Gly-Glu-Pro-Arg-Gly-Asp-Thr-Tyr-Arg-Ala-Tyr-NH₂ (seq. ID No.: 2), respectively.

The microcarrier of the disclosure can be, if desired, advantageously functionalized by simple physical adsorption of polymers such as adhesive peptides.

Suitable polymers promoting cell adhesion may comprise a synthetic polymer. Examples of such a synthetic polymer bearing peptide that promotes cell adhesion and growth includes Corning Incorporated's Synthemax^{®}II. Eliminating chemical derivatization from the manufacturing process by using physical adsorption of an adhesion promoting polymer appears attractive since chemical derivatization is time consuming, labor intensive, requires a large amount of reagents, and generates a large amount of waste chemicals.

The coating prepared from polymers comprising adhesive peptides is particularly effective when performed on beads that have been cross-linked by internal gelation.

Depending of the digestion time, temperature, and amount of chelating agent or enzyme added, the extent of digestion of the microcarrier beads can be selected or predetermined. In some cases, cells can detach from the surface before the whole bead is fully digested, so that it is possible to harvest the cells without complete digestion of the beads or after complete bead digestion. In the former, the beads must be separated from the cells by means of a physical process, e.g., filtration, decantation, centrifugation, and like processing, or combinations thereof.

The host cell cultures of the present disclosure may be cultured in a suspension medium, such as a chemically-defined medium or serum free medium.

## Claims

1. A method of culturing adherent cells in suspension, comprising:
culturing adherent cells on a first substrate in a first suspension, wherein the first substrate comprises a first dissolvable microcarrier;
harvesting adherent cells from the first substrate;
transfecting the harvested adherent cells using electroporation;
after the step of transfecting, suspending the transfected adherent cells in a second suspension, wherein the transfected adherent cells are suspended in the second suspension on a second substrate and wherein the second substrate comprises a second dissolvable microcarrier; and
harvesting the cells or products of the cells from the second suspension, the harvesting comprising a dissolution process for dissolving the second dissolvable microcarrier to harvest the cells or cell products.

2. The method of claim 1, wherein the dissolution process comprises:
adding a chelator to the second suspension;
contacting the cultured cells with the chelator for a predetermined time to separate the cells from the second microcarrier; and
isolating the cells or cell products from a remainder of the second suspension after the predetermined time.

3. The method of claim 2, wherein the chelator is ethylenediamine tetraacetic acid (EDTA).

4. The method of claim 2 or claim 3, wherein the isolating comprises centrifuging or filtering the second suspension to separate the cells or cell products from the remainder of the second suspension.

5. The method of any one of claims 2-4, further comprising performing lysis of the isolated cells or cell products.

6. The method of any one of claims 2-5, wherein the contacting and the separation of the cells from the second microcarrier are performed free of a protease, a pectinase, or both a protease and a pectinase.

7. The method of any one of claims 1-6 wherein the dissolution process does not include a washing step.

8. The method of any one of claims 1-7, wherein the dissolution process does not include a phosphate-buffered saline wash.

9. The method of any one of claims 2-6, wherein the predetermined time is from about 1 minute to about 30 minutes; is from about 5 minutes to about 20 minutes, is from about 8 minutes to about 12 minutes, or is at least about 10 minutes.

## Patentansprüche

1. Verfahren zum Kultivieren von adhärenten Zellen in Suspension, umfassend:
Kultivieren von adhärenten Zellen auf einem ersten Substrat in einer ersten Suspension, wobei das erste Substrat einen ersten auflösbaren Mikroträger umfasst;
Ernten von adhärenten Zellen von dem ersten Substrat;
Transfizieren der geernteten adhärenten Zellen unter Verwendung von Elektroporation;
nach dem Schritt des Transfizierens Suspendieren der transfizierten adhärenten Zellen in einer zweiten Suspension, wobei die transfizierten adhärenten Zellen in der zweiten Suspension auf einem zweiten Substrat suspendiert sind und wobei das zweite Substrat einen zweiten auflösbaren Mikroträger umfasst; und
Ernten der Zellen oder Produkte der Zellen aus der zweiten Suspension, wobei das Ernten einen Auflösungsvorgang zum Auflösen des zweiten auflösbaren Mikroträgers umfasst, um die Zellen oder Zellprodukte zu ernten.

2. Verfahren nach Anspruch 1, wobei der Auflösungsvorgang Folgendes umfasst:
Hinzufügen eines Chelatbildners zu der zweiten Suspension;
Kontaktieren der kultivierten Zellen mit dem Chelatbildner für eine vorbestimmte Zeit, um die Zellen von dem zweiten Mikroträger zu trennen; und
Isolieren der Zellen oder Zellprodukte aus einem Rest der zweiten Suspension nach der vorbestimmten Zeit.

3. Verfahren nach Anspruch 2, wobei der Chelatbildner Ethylendiamintetraessigsäure (EDTA) ist.

4. Verfahren nach Anspruch 2 oder Anspruch 3, wobei das Isolieren Zentrifugieren oder Filtrieren der zweiten Suspension umfasst, um die Zellen oder Zellprodukte von dem Rest der zweiten Suspension zu trennen.

5. Verfahren nach einem der Ansprüche 2-4, ferner umfassend Durchführen einer Lyse der isolierten Zellen oder Zellprodukte.

6. Verfahren nach einem der Ansprüche 2-5, wobei das Kontaktieren und die Trennung der Zellen von dem zweiten Mikroträger frei von einer Protease, einer Pektinase oder sowohl einer Protease als auch einer Pektinase durchgeführt werden.

7. Verfahren nach einem der Ansprüche 1-6, wobei der Auflösungsvorgang keinen Waschschritt einschließt.

8. Verfahren nach einem der Ansprüche 1-7, wobei der Auflösungsvorgang keine Waschung mit phosphatgepufferter Salzlösung einschließt.

9. Verfahren nach einem der Ansprüche 2-6, wobei die vorbestimmte Zeit von etwa 1 Minute bis etwa 30 Minuten beträgt; von etwa 5 Minuten bis etwa 20 Minuten beträgt, von etwa 8 Minuten bis etwa 12 Minuten beträgt oder mindestens etwa 10 Minuten beträgt.

## Revendications

1. Procédé de culture de cellules adhérentes en suspension, comprenant :
la culture de cellules adhérentes sur un premier substrat dans une première suspension, dans lequel le premier substrat comprend une première microporteuse soluble ;
la récolte de cellules adhérentes à partir du premier substrat ;
la transfection des cellules adhérentes récoltées à l'aide d'électroporation ;
après l'étape de transfection, la mise en suspension des cellules adhérentes transfectées dans une seconde suspension, dans lequel les cellules adhérentes transfectées sont mises en suspension dans la seconde suspension sur un second substrat et dans lequel le second substrat comprend une seconde microporteuse soluble ; et
la récolte des cellules ou de produits des cellules à partir de la seconde suspension, la récolte comprenant un processus de dissolution destiné à dissoudre la seconde microporteuse soluble pour récolter les cellules ou les produits de cellules.

2. Procédé de la revendication 1, dans lequel le processus de dissolution comprend :
l'ajout d'un chélatant à la seconde suspension ;
la mise en contact des cellules cultivées avec le chélatant pendant une durée prédéterminée pour séparer les cellules de la seconde microporteuse ; et
l'isolation des cellules ou des produits de cellules d'un reste de la seconde suspension après la durée prédéterminée.

3. Procédé de la revendication 2, dans lequel le chélatant est l'acide éthylènediamine tétraacétique (EDTA).

4. Procédé de la revendication 2 ou de la revendication 3, dans lequel l'isolation comprend la centrifugation ou la filtration de la seconde suspension pour séparer les cellules ou les produits de cellules du reste de la seconde suspension.

5. Procédé de l'une quelconque des revendications 2 à 4, comprenant en outre la réalisation d'une lyse des cellules ou des produits de cellules isolés.

6. Procédé de l'une quelconque des revendications 2 à 5, dans lequel la mise en contact et la séparation des cellules de la seconde microporteuse sont réalisées sans une protéase, sans une pectinase, ou sans une protéase ni une pectinase.

7. Procédé de l'une quelconque des revendications 1 à 6, dans lequel le processus de dissolution ne comprend pas d'étape de lavage.

8. Procédé de l'une quelconque des revendications 1 à 7, dans lequel le processus de dissolution ne comprend pas de lavage salin tamponné au phosphate.

9. Procédé de l'une quelconque des revendications 2 à 6, dans lequel la durée prédéterminée est d'environ 1 minute à environ 30 minutes ; est d'environ 5 minutes à environ 20 minutes, est d'environ 8 minutes à environ 12 minutes, ou est d'au moins environ 10 minutes.
